# EUROPEAN PATENT APPLICATION

(11) **EP 4 220 656 A1**
(43) Date of publication of application: **02.08.2023**
(21) Application number: 21871970.6
(22) Date of filing: 29.07.2021
(51) Int. Cl.: G16H 30/20, G16H 50/20, A61B 5/00

(54) **INFORMATION PROCESSING SYSTEM, INFORMATION PROCESSING METHOD, AND INFORMATION PROCESSING PROGRAM**

(30) Priority: 28.09.2020 JP 2020162701
(71) Applicant: FUJIFILM Corporation, Tokyo 106-8620 (JP)
(72) Inventor: MORITA, Masaharu, Tokyo 106-8620 (JP)
(74) Representative: Klunker IP Patentanwälte PartG mbB
(86) International application number: PCT/JP2021/028137
(87) International publication number: WO 2022/064838

(57) **Abstract**

An information processing system, an information processing method, and an information processing program capable of improving the efficiency of diagnosis using a CAD result performed by three parties, that is, an imaging technician, an image interpreter, and a diagnostician. A first information processing device transmits an execution result of lesion detection processing executed on a medical image, a second information processing device performs control to display the medical image and the execution result and transmits the first input information of the imaging technician for the execution result, a third information processing device performs control to display the medical image, the execution result, and the first input information and transmits the second input information of the image interpreter for the execution result, and a fourth information processing device performs control to display the medical image, the execution result, and the second input information and stores a comprehensive diagnosis result of the diagnostician.

## Description

### Technical Field

The present disclosure relates to an information processing system, an information processing method, and an information processing program.

### Related Art

Conventionally, in the medical field, a computer aided diagnosis (CAD) system that supports an image diagnosis performed by a person by detecting a lesion in an image through a computer is known. Hereinafter, a detection result obtained by performing lesion detection processing on a medical image through the computer will be referred to as a "CAD result".

As a technology related to a CAD system, JP1994-251038A (JP-H06-251038A) discloses a medical diagnosis support system that acquires first diagnosis information by performing computer processing on medical examination data and that acquires second diagnosis information obtained through image interpretation of the medical examination data performed by an image interpreter, such as a doctor. In a case where a processing target range of the first diagnosis information is not an entire range of the medical examination data, the medical diagnosis support system determines whether or not a target range of the second diagnosis information is within the processing target range of the first diagnosis information.

Further, JP2011-103095A discloses a medical image display system that acquires examination result information for a medical image displayed on a display device, and a medical image of a past examination in the same patient and the same examination conditions as the medical image and detection result information obtained by a computer for the medical image. This medical image display system extracts, on the basis of the acquired detection result information, an image of a portion of an abnormal shadow candidate included in the medical image being displayed on the display device and an image of a portion of an abnormal shadow candidate included in the medical image of the past examination, and displays a list of extracted images in a time series on the display device.

Further, JP2004-216008A discloses an image diagnosis support device that detects abnormal shadow candidates from medical images and that decides a display order of the medical images on the basis of malignancy, contrast, diagnosis difficulty, patient information, or an imaging site of a detected abnormal shadow candidate portion.

### SUMMARY OF THE INVENTION

### Technical Problem

In the work of diagnosing a patient using a medical image, each of an imaging technician of a medical image, such as a radiographer, an medical image interpreter, such as an image interpretation doctor, and a diagnostician who performs a comprehensive diagnosis of the patient, such as a general diagnostician, interprets the medical image with reference to a CAD result, in many cases. In this case, in a case where the current policies of "the CAD result is a support of an image diagnosis performed by a person" and "the person has the ultimate responsibility for the diagnosis" are operated, the loads on the imaging technician, the image interpreter, and the diagnostician are not reduced even with a diagnosis support using CAD.

In recent years, the accuracy of the CAD result has improved, and the frequency of erroneous detection of a lesion has decreased. In addition, even if the CAD result includes erroneous detection of a lesion, a risk to the life of the patient is low in a case where the error is closer to a safe side. On the other hand, in a case where the CAD result is that the lesion is not detected, there is a risk for the patient that the lesion is overlooked.

In addition, in a case where CAD is designed such that a computer detects even a very small lesion for a specific type of lesion, the number of detected lesions increases, which is troublesome for a person who interprets an image. In that respect, there is also a facility that has an operation in which a person observes very small lesions and a computer does not actively detect the very small lesions. For example, there is an operation in which a tumor of 5 mm or less in a lung disease is excluded from the detection target of the CAD.

Under the above assumptions, it is considered that, if there is a system capable of realizing a flow in which the imaging technician, the image interpreter, and the diagnostician perform only a simple confirmation for the lesion detected by the CAD and intensively confirm a region in which nothing is detected in the medical image by the CAD, it is possible to improve the efficiency of diagnosis using the CAD result performed by three parties, that is, the imaging technician, the image interpreter, and the diagnostician.

The technologies disclosed in JP1994-251038A(JP-H06-251038A), JP2011-103095A, and JP2004-216008A improve the efficiency of image interpretation in a case where the image interpreter who interprets the medical image performs image interpretation with reference to the CAD result, but do not improve the efficiency of diagnosis performed by the three parties, that is, the imaging technician, the image interpreter, and the diagnostician.

### Solution to Problem

The present disclosure has been made in view of the above circumstances, and an object of the present disclosure is to provide an information processing system, an information processing method, and an information processing program capable of improving the efficiency of diagnosis using a CAD result performed by three parties, that is, an imaging technician, an image interpreter, and a diagnostician.

According to the present disclosure, there is provided an information processing system comprising: a first information processing device for diagnosis of a medical image performed by a computer; a second information processing device for an imaging technician who captures the medical image; a third information processing device for an image interpreter who interprets the medical image; and a fourth information processing device for a diagnostician who performs a comprehensive diagnosis of a patient, in which each of the first information processing device, the second information processing device, the third information processing device, and the fourth information processing device includes at least one processor, the processor of the first information processing device is configured to acquire a medical image to be diagnosed, execute lesion detection processing on the acquired medical image, and transmit an execution result of the lesion detection processing, the processor of the second information processing device is configured to perform control to display the medical image and the execution result, receive first input information of the imaging technician for the execution result, and transmit the first input information, the processor of the third information processing device is configured to perform control to display the medical image, the execution result, and the first input information, receive second input information of the image interpreter for the execution result, and transmit the second input information, and the processor of the fourth information processing device is configured to perform control to display the medical image, the execution result, and the second input information, receive a comprehensive diagnosis result of the diagnostician, and store the diagnosis result.

In the information processing system of the present disclosure, the first input information may be information indicating whether to approve or disapprove the execution result.

In addition, in the information processing system of the present disclosure, the first input information may be information indicating a non-detected lesion that is not included in the execution result.

Further, in the information processing system of the present disclosure, the first input information may be information indicating deletion of a lesion included in the execution result.

In addition, in the information processing system of the present disclosure, the second input information may be information indicating whether to approve or disapprove the execution result and the first input information.

Further, in the information processing system of the present disclosure, the second input information may be information indicating a non-detected lesion that is not included in the execution result and in the first input information.

Further, in the information processing system of the present disclosure, the processor of the fourth information processing device may be configured to perform control to display the first input information in addition to the medical image and the second input information.

Further, in the information processing system of the present disclosure, there may be a plurality of the medical images to be diagnosed, and the processors of the second information processing device, of the third information processing device, and of the fourth information processing device may be configured to perform, in a case where the execution result includes information indicating one or more lesions, control to display the medical image in a state in which the number of medical images is reduced, when displaying the medical image to be diagnosed.

In addition, according to the present disclosure, there is provided an information processing method using an information processing system including a first information processing device for diagnosis of a medical image performed by a computer, a second information processing device for an imaging technician who captures the medical image, a third information processing device for an image interpreter who interprets the medical image, and a fourth information processing device for a diagnostician who performs a comprehensive diagnosis of a patient, the information processing method comprising: causing a processor provided in the first information processing device to acquire a medical image to be diagnosed, execute lesion detection processing on the acquired medical image, and transmit an execution result of the lesion detection processing; causing a processor provided in the second information processing device to perform control to display the medical image and the execution result, receive first input information of the imaging technician for the execution result, and transmit the first input information; causing a processor provided in the third information processing device to perform control to display the medical image, the execution result, and the first input information, receive second input information of the image interpreter for the execution result, and transmit the second input information; and causing a processor provided in the fourth information processing device to perform control to display the medical image, the execution result, and the second input information, receive a comprehensive diagnosis result of the diagnostician, and store the diagnosis result.

Further, according to the present disclosure, there is provided an information processing program in an information processing system including a first information processing device for diagnosis of a medical image performed by a computer, a second information processing device for an imaging technician who captures the medical image, a third information processing device for an image interpreter who interprets the medical image, and a fourth information processing device for a diagnostician who performs a comprehensive diagnosis of a patient, the information processing program causing a processor provided in the first information processing device to execute processing of: acquiring a medical image to be diagnosed; executing lesion detection processing on the acquired medical image; and transmitting an execution result of the lesion detection processing, causing a processor provided in the second information processing device to execute processing of: performing control to display the medical image and the execution result; receiving first input information of the imaging technician for the execution result; and transmitting the first input information, causing a processor provided in the third information processing device to execute processing of: performing control to display the medical image, the execution result, and the first input information; receiving second input information of the image interpreter for the execution result; and transmitting the second input information, and causing a processor provided in a processor of the fourth information processing device to execute processing of: performing control to display the medical image, the execution result, and the second input information; receiving a comprehensive diagnosis result of the diagnostician; and storing the diagnosis result.

### Effects of Invention

According to the present disclosure, it is possible to improve the efficiency of diagnosis using a CAD result performed by three parties, that is, an imaging technician, an image interpreter, and a diagnostician.

### BRIEF DESCRIPTION OF THE DRAWINGS

Fig. 1 is a block diagram showing an example of a configuration of an information processing system.
Fig. 2 is a block diagram showing an example of a hardware configuration of an information processing device.
Fig. 3 is a block diagram showing an example of a functional configuration of a first information processing device.
Fig. 4 is a block diagram showing an example of a functional configuration of a second information processing device.
Fig. 5 is a diagram showing an example of a display screen of the second information processing device.
Fig. 6 is a block diagram showing an example of a functional configuration of a third information processing device.
Fig. 7 is a diagram showing an example of a display screen of the third information processing device.
Fig. 8 is a block diagram showing an example of a functional configuration of a fourth information processing device.
Fig. 9 is a diagram showing an example of a display screen of the fourth information processing device.
Fig. 10 is a flowchart showing an example of a first diagnosis support processing.
Fig. 11 is a flowchart showing an example of a second diagnosis support processing.
Fig. 12 is a flowchart showing an example of a third diagnosis support processing.
Fig. 13 is a flowchart showing an example of a fourth diagnosis support processing.
Fig. 14 is a diagram showing an example of a display screen of a second information processing device according to a modification example.

### DESCRIPTION OF EMBODIMENTS

Hereinafter, an example of an embodiment of the technology of the present disclosure will be described in detail with reference to the drawings.

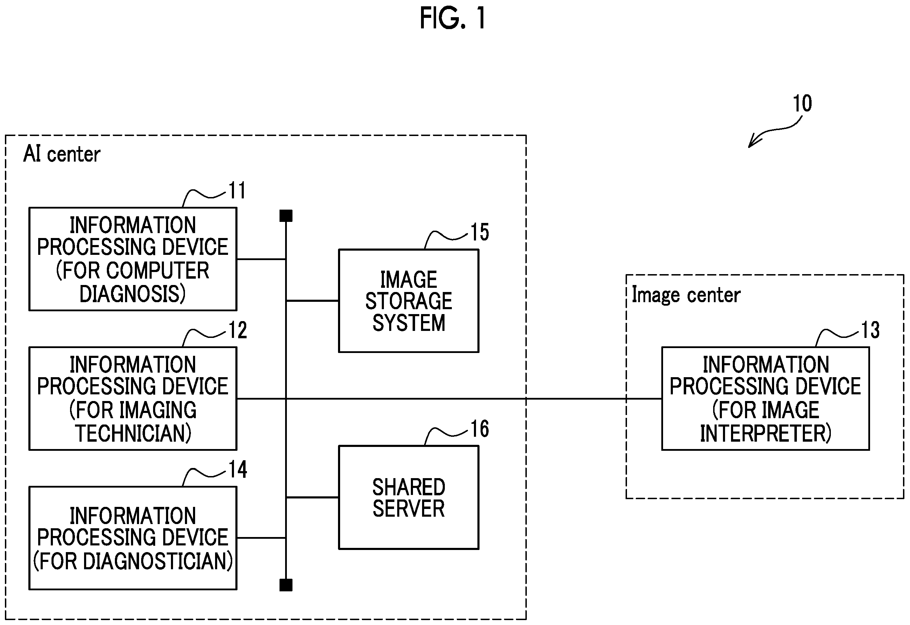

First, a configuration of an information processing system 10 according to the present embodiment will be described with reference to Fig. 1. As shown in Fig. 1, the information processing system 10 includes an information processing device 11, an information processing device 12, an information processing device 13, an information processing device 14, an image storage system 15, and a shared server 16. The information processing device 11, the information processing device 12, the information processing device 14, the image storage system 15, and the shared server 16 are installed in a facility called an Artificial Intelligence (AI) center, which mainly performs diagnosis of medical images through a computer. The information processing device 13 is installed in a facility called an image center, which mainly interprets medical images. The information processing device 11, the information processing device 12, the information processing device 13, the information processing device 14, the image storage system 15, and the shared server 16 are each connected to a network and can communicate with each other.

The information processing device 11 is a first information processing device for diagnosis of a medical image performed by a computer, such as CAD. The information processing device 12 is a second information processing device for an imaging technician who captures the medical image, such as a radiographer. The information processing device 13 is a third information processing device for an image interpreter who interprets the medical image, such as an image interpretation doctor. The information processing device 14 is a fourth information processing device for a diagnostician who performs a comprehensive diagnosis of a patient, such as a general diagnostician. Examples of the information processing devices 11 to 14 include a computer, such as a personal computer or a server computer.

The image storage system 15 is a system that stores image data showing a medical image captured by an imaging device which captures the medical image. The image storage system 15 transmits the image data corresponding to requests from the information processing devices 11 to 14 and the like to the request source device. Examples of the image storage system 15 include a Picture Archiving and Communication Systems (PACS). Examples of medical images include a CT image captured by a Computed Tomography (CT) device, an MRI image captured by Magnetic Resonance Imaging (MRI), a radiation image captured by a Flat Panel Detector (FPD), and an endoscopic image captured by an endoscope system. In the present embodiment, an example in which a plurality of tomographic images captured by a CT device are applied as medical images to be diagnosed will be described.

The shared server 16 has a storage device that stores information added by the information processing devices 11 to 14 to the medical image. The medical image stored in the image storage system 15 and the information added to the medical image and stored in the shared server 16 are associated with each other by, for example, identification information, such as a patient IDentifier (ID) and an examination ID.

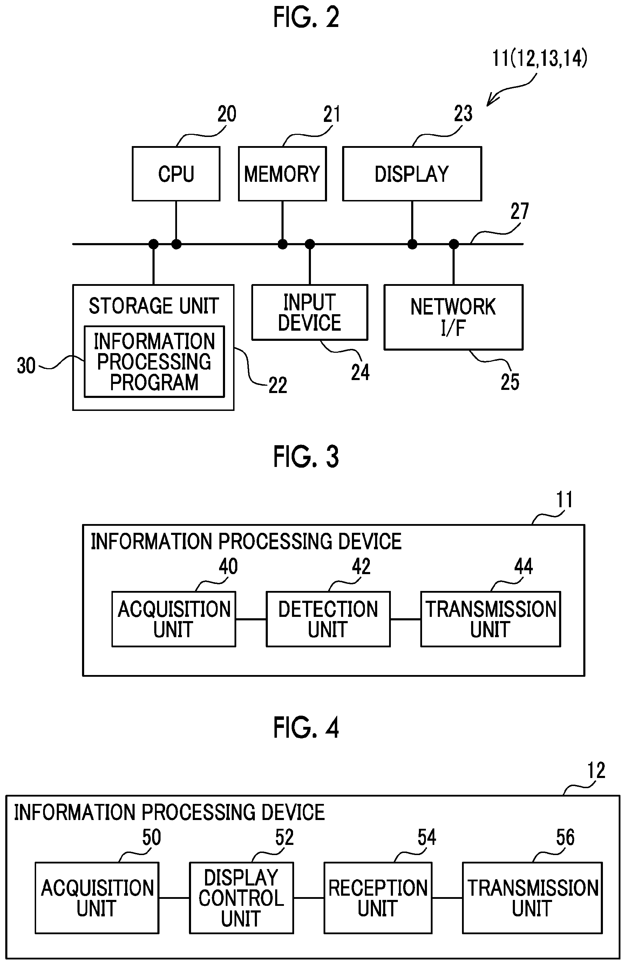

Next, a hardware configuration of the information processing device 11 according to the present embodiment will be described with reference to Fig. 2. Since hardware configurations of the information processing devices 12 to 14 are the same as the hardware configuration of the information processing device 11, the description thereof will be omitted. As shown in Fig. 2, the information processing device 11 includes a central processing unit (CPU) 20, a memory 21 serving as a temporary storage area, and a non-volatile storage unit 22. In addition, the information processing device 11 includes a display 23, such as a liquid crystal display, an input device 24, such as a keyboard and a mouse, and a network interface (I/F) 25 connected to a network. The CPU 20, the memory 21, the storage unit 22, the display 23, the input device 24, and the network I/F 25 are connected to a bus 27.

The storage unit 22 is realized by a hard disk drive (HDD), a solid state drive (SSD), a flash memory, or the like. An information processing program 30 is stored in the storage unit 22 serving as a storage medium. The CPU 20 reads out the information processing program 30 from the storage unit 22 and then develops the read-out information processing program 30 into the memory 21, and executes the developed information processing program 30.

Next, a functional configuration of the information processing device 11 according to the present embodiment will be described with reference to Fig. 3. As shown in Fig. 3, the information processing device 11 includes an acquisition unit 40, a detection unit 42, and a transmission unit 44. The CPU 20 of the information processing device 11 executes the information processing program 30 to function as the acquisition unit 40, the detection unit 42, and the transmission unit 44.

The acquisition unit 40 acquires the medical image to be diagnosed from the image storage system 15 via the network I/F 25. The detection unit 42 executes lesion detection processing on the medical image acquired by the acquisition unit 40. For example, the detection unit 42 executes the lesion detection processing on the medical image by using a known CAD lesion detection algorithm. This algorithm is prepared in advance according to, for example, an imaging site and a type of lesion to be detected. In addition, in the lesion detection processing according to the present embodiment, a lesion having a size equal to or larger than a threshold value set in advance is detected. In this case, the threshold value is set in advance according to, for example, the imaging site and the type of lesion to be detected.

The detection unit 42 may execute the lesion detection processing on the medical image by using a detection model obtained by machine learning, such as deep learning. Alternatively, the detection unit 42 may execute the lesion detection processing on the medical image through filtering processing using a filter for detecting the lesion.

The transmission unit 44 transmits an execution result of the lesion detection processing performed by the detection unit 42 (hereinafter, referred to as "lesion detection result") to the shared server 16 via the network I/F 25. The shared server 16 stores the lesion detection result transmitted from the information processing device 11. In a case where a lesion is detected by the detection unit 42, the lesion detection result includes information indicating the detected lesion. Examples of the information indicating the lesion include a type of the lesion, a position of the lesion in the medical image, and a size of the lesion. On the other hand, in a case where the lesion is not detected by the detection unit 42, the lesion detection result includes information indicating that the lesion is not detected. The transmission unit 44 may transmit the lesion detection result to the information processing device 12 via the network I/F 25.

Next, a functional configuration of the information processing device 12 according to the present embodiment will be described with reference to Fig. 4. As shown in Fig. 4, the information processing device 12 includes an acquisition unit 50, a display control unit 52, a reception unit 54, and a transmission unit 56. The CPU 20 of the information processing device 12 executes the information processing program 30 to function as the acquisition unit 50, the display control unit 52, the reception unit 54, and the transmission unit 56.

The acquisition unit 50 acquires the medical image to be diagnosed from the image storage system 15 via the network I/F 25. In addition, the acquisition unit 50 acquires the lesion detection result corresponding to the medical image to be diagnosed from the shared server 16 via the network I/F 25.

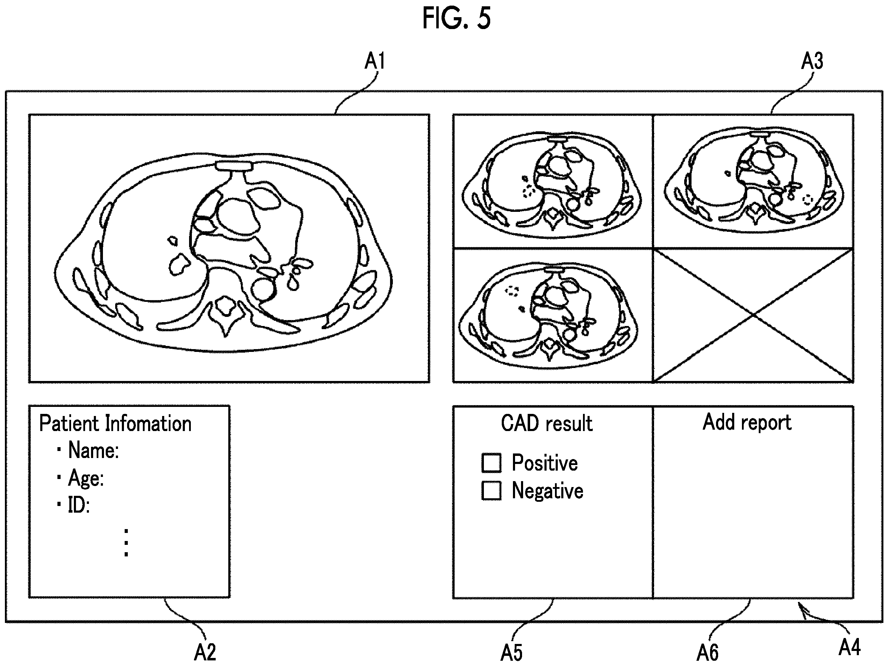

The display control unit 52 performs control to display the medical image and the lesion detection result, which are acquired by the acquisition unit 50, on the display 23. Fig. 5 shows an example of a first display screen displayed on the display 23 of the information processing device 12 by this control.

As shown in Fig. 5, the first display screen includes a display region A1 for a medical image, a display region A2 for patient information, a display region A3 for a lesion detection result, and a display region A4 for an input field of the first input information of the imaging technician. The medical image to be diagnosed acquired by the acquisition unit 50 is displayed on the display region A1. A tomographic position of the medical image first displayed on the display region A1 is set in advance, and the imaging technician can switch the medical image displayed on the display region A1 by scrolling a mouse wheel or the like.

Patient information, such as the name, the age, and the ID of the patient, is displayed on the display region A2. In a case where a lesion is detected by the information processing device 11, the lesion is displayed on the display region A3 in a distinguishable manner. In this case, for example, the medical image in which the lesion is detected is displayed on the display region A3 in a state in which a frame line of the detected lesion is colored. Fig. 5 shows an example in which lesions are detected from three medical images and frame lines of the detected lesions are indicated by broken lines. Further, in a case where a lesion is not detected by the information processing device 11, information indicating that no lesion is detected is displayed on the display region A3. In this case, for example, a message, such as "No lesion is detected" is displayed on the display region A3.

The display region A4 includes a display region A5 for an input field of information indicating whether to approve or disapprove the lesion detection result, and a display region A6 for an input field of an additional report. In a case where the imaging technician approves the lesion detection result by referring to the display region A3, the imaging technician inputs information indicating approval of the lesion detection result by putting a checkmark in a "Positive" check box on the display region A5 via the input device 24. On the other hand, in a case where the imaging technician disapproves the lesion detection result by referring to the display region A3, the imaging technician inputs information indicating disapproval of the lesion detection result by putting a checkmark in a "Negative" check box on the display region A5 via the input device 24. This information input via the display region A5 is an example of the first input information.

Further, in a case where a non-detected lesion which is not included in the lesion detection result is found in the medical image, the imaging technician inputs information indicating the non-detected lesion as the first input information via the input device 24. For example, the imaging technician traces an outer frame of the found lesion in the medical image displayed on the display region A1 to input information indicating the position and the size of the lesion, and inputs information indicating the type of the lesion into the display region A6. In addition, in a case where there is additional information to be input in the display region A6, the imaging technician inputs the information.

The reception unit 54 receives the first input information of the imaging technician for the lesion detection result input as described above. The transmission unit 56 transmits the first input information received by the reception unit 54 to the shared server 16 via the network I/F 25. The shared server 16 stores the first input information transmitted from the information processing device 12. The transmission unit 56 may transmit the first input information to the information processing device 13 via the network I/F 25.

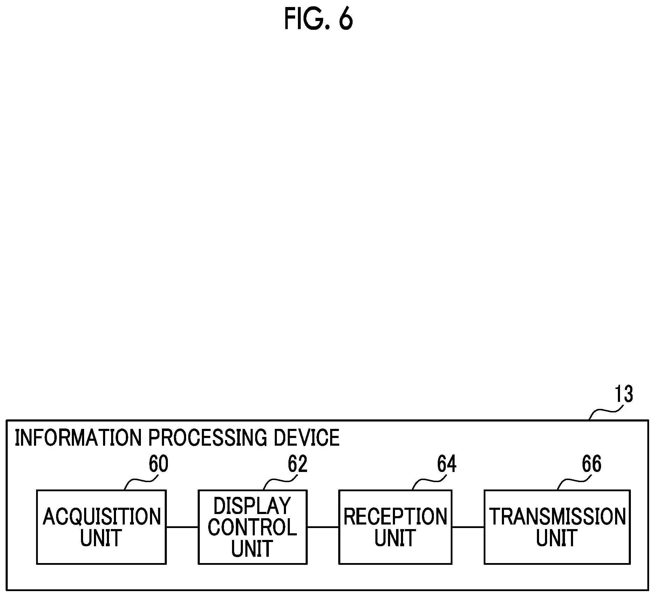

Next, a functional configuration of the information processing device 13 according to the present embodiment will be described with reference to Fig. 6. As shown in Fig. 6, the information processing device 13 includes an acquisition unit 60, a display control unit 62, a reception unit 64, and a transmission unit 66. The CPU 20 of the information processing device 13 executes the information processing program 30 to function as the acquisition unit 60, the display control unit 62, the reception unit 64, and the transmission unit 66.

The acquisition unit 60 acquires the medical image to be diagnosed from the image storage system 15 via the network I/F 25. In addition, the acquisition unit 60 acquires the lesion detection result and the first input information corresponding to the medical image to be diagnosed from the shared server 16 via the network I/F 25.

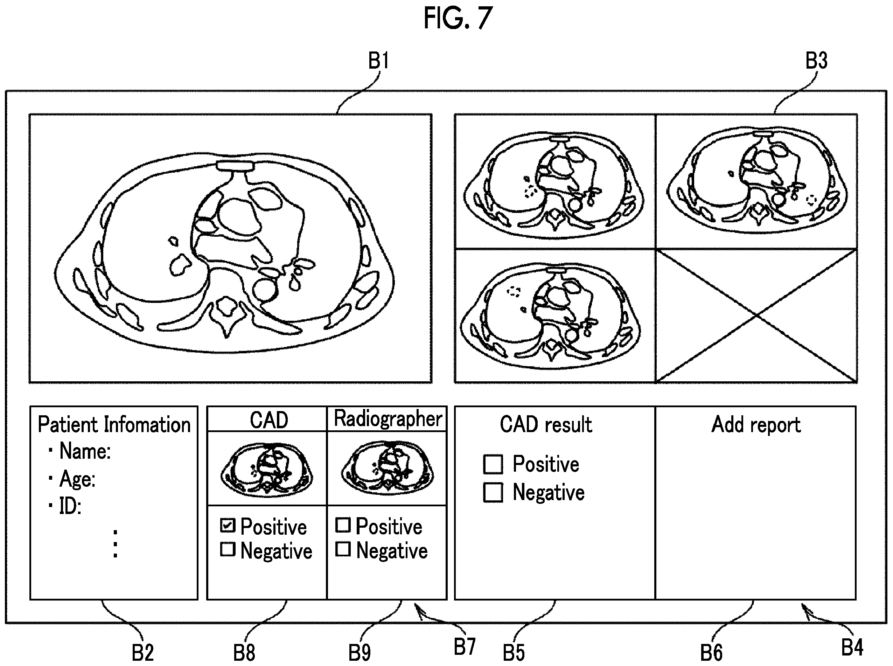

The display control unit 62 performs control to display the medical image, the lesion detection result, and the first input information, which are acquired by the acquisition unit 60, on the display 23. Fig. 7 shows an example of a second display screen displayed on the display 23 of the information processing device 13 by this control.

As shown in Fig. 7, the second display screen includes a display region B1 for a medical image, a display region B2 for patient information, a display region B3 for a lesion detection result, a display region B4 for an input field of the second input information of the image interpreter, and a display region B7 for the first input information. The display region B4 includes a display region B5 for an input field of information indicating whether to approve or disapprove the lesion detection result, and a display region B6 for an input field of an additional report. Since the display regions B1 to B6 are the same display regions as the display regions A1 to A6, the description thereof will be omitted here.

The display region B7 includes a display region B8 for information indicating whether the imaging technician has approved or disapproved the lesion detection result, and a display region B9 for a lesion that is not included in the lesion detection result and that is found and input as the first input information by the imaging technician. Fig. 7 shows an example in which the lesion detection result is displayed in an upper part of the display region B8 and a checkmark is put on "Positive" in a lower part, that is, an example in which the imaging technician has approved the lesion detection result. Further, in the example of Fig. 7, the lesion found by the imaging technician is displayed in an upper part of the display region B9, and an input field to which information indicating whether the image interpreter approves or disapproves the lesion found by the imaging technician is input is provided in a lower part.

In a case where the image interpreter approves the lesion detection result by referring to the display region B3, the image interpreter inputs information indicating approval of the lesion detection result by putting a checkmark in a "Positive" check box on the display region B5 via the input device 24. On the other hand, in a case where the image interpreter disapproves the lesion detection result by referring to the display region B3, the image interpreter inputs information indicating disapproval of the lesion detection result by putting a checkmark in a "Negative" check box on the display region B5 via the input device 24. This information input via the display region B5 is an example of the second input information.

Further, in a case where a non-detected lesion which is not included in the execution result and in the first input information is found in the medical image, the image interpreter inputs information indicating the non-detected lesion as the second input information via the input device 24. For example, the image interpreter traces an outer frame of the found lesion in the medical image displayed on the display region B1 to input information indicating the position and the size of the lesion, and inputs information indicating the type of the lesion into the display region B6. In addition, in a case where there is additional information to be input in the display region B6, the image interpreter inputs the information.

In addition, in a case where the image interpreter approves the lesion found by the imaging technician and included in the first input information, the image interpreter inputs information indicating approval of the first input information by putting a checkmark in a "Positive" check box on the display region B9 via the input device 24. On the other hand, in a case where the image interpreter disapproves the lesion found by the imaging technician and included in the first input information, the image interpreter inputs information indicating disapproval of the first input information by putting a checkmark in a "Negative" check box on the display region B9 via the input device 24. This information input via the display region B9 is also an example of the second input information.

The reception unit 64 receives the lesion detection result and the second input information of the image interpreter for the first input information, which are input as described above. The transmission unit 66 transmits the second input information received by the reception unit 64 to the shared server 16 via the network I/F 25. The shared server 16 stores the second input information transmitted from the information processing device 13. The transmission unit 66 may transmit the second input information to the information processing device 14 via the network I/F 25.

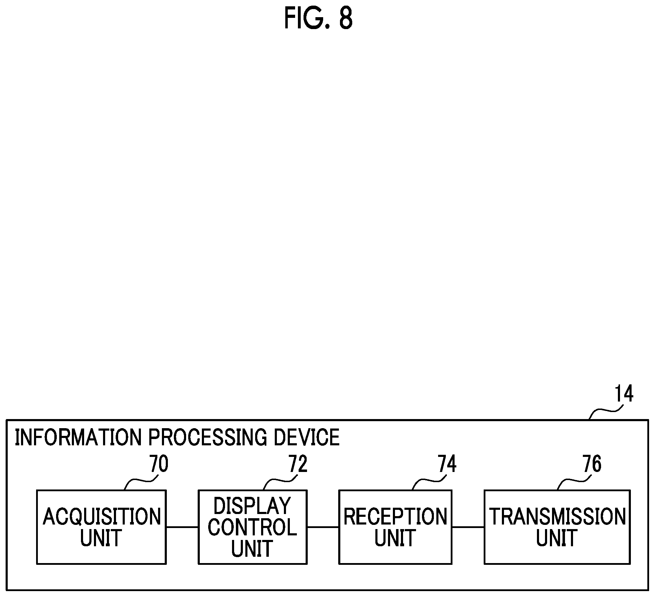

Next, a functional configuration of the information processing device 14 according to the present embodiment will be described with reference to Fig. 8. As shown in Fig. 8, the information processing device 14 includes an acquisition unit 70, a display control unit 72, a reception unit 74, and a transmission unit 76. The CPU 20 of the information processing device 14 executes the information processing program 30 to function as the acquisition unit 70, the display control unit 72, the reception unit 74, and the transmission unit 76.

The acquisition unit 70 acquires the medical image to be diagnosed from the image storage system 15 via the network I/F 25. In addition, the acquisition unit 70 acquires the lesion detection result and the second input information corresponding to the medical image to be diagnosed from the shared server 16 via the network I/F 25.

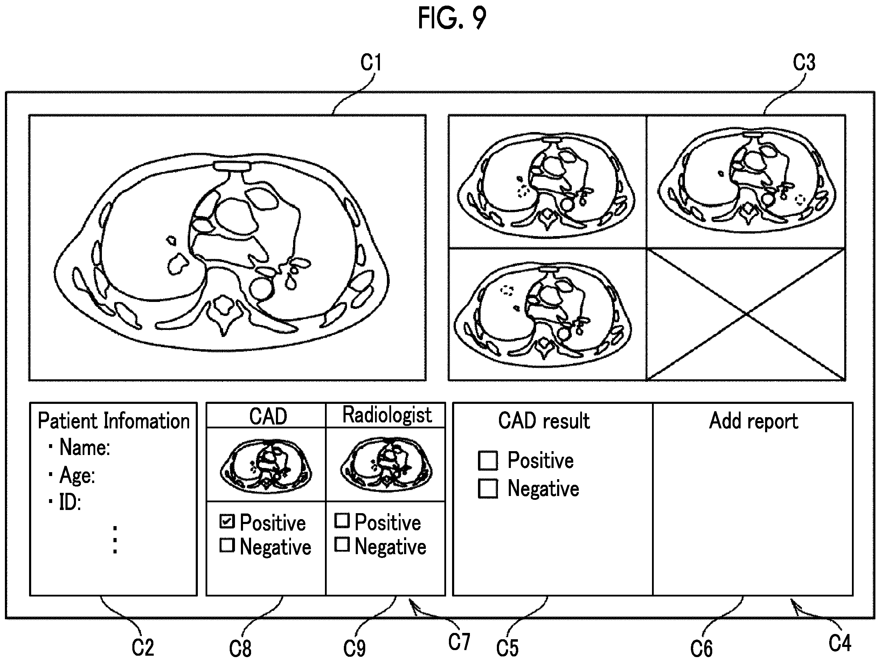

The display control unit 72 performs control to display the medical image, the lesion detection result, and the second input information, which are acquired by the acquisition unit 70, on the display 23. Fig. 9 shows an example of a third display screen displayed on the display 23 of the information processing device 14 by this control.

As shown in Fig. 9, the third display screen includes a display region C1 for a medical image, a display region C2 for patient information, a display region C3 for a lesion detection result, a display region C4 for an input field of the comprehensive diagnosis result of the diagnostician, and a display region C7 for the second input information. The display region C4 includes a display region C5 for an input field of information indicating whether to approve or disapprove the lesion detection result, and a display region C6 for an input field of an additional report. Since the display regions C1 to C6 are the same display regions as the display regions B 1 to B6, the description thereof will be omitted here.

The display region C7 includes a display region C8 for information indicating whether the image interpreter has approved or disapproved the lesion detection result, and a display region C9 for a lesion that is not included in the lesion detection result and that is found and input as the second input information by the image interpreter. Fig. 9 shows an example in which the lesion detection result is displayed in an upper part of the display region C8 and a checkmark is put on "Positive" in a lower part, that is, an example in which the image interpreter has approved the lesion detection result. Further, in the example of Fig. 9, the lesion found by the image interpreter is displayed in an upper part of the display region C9, and an input field to which information indicating whether the diagnostician approves or disapproves the lesion found by the image interpreter is input is provided in a lower part.

In a case where the diagnostician approves the lesion detection result by referring to the display region C3, the diagnostician inputs information indicating approval of the lesion detection result by putting a checkmark in a "Positive" check box on the display region C5 via the input device 24. On the other hand, in a case where the diagnostician disapproves the lesion detection result by referring to the display region C3, the diagnostician inputs information indicating disapproval of the lesion detection result by putting a checkmark in a "Negative" check box on the display region C5 via the input device 24.

Further, in a case where a non-detected lesion which is not included in the execution result and in the second input information is found in the medical image, the diagnostician inputs information indicating the non-detected lesion via the input device 24. For example, the diagnostician traces an outer frame of the found lesion in the medical image displayed on the display region C1 to input information indicating the position and the size of the lesion, and inputs information indicating the type of the lesion into the display region C6.

In addition, in a case where the diagnostician approves the lesion found by the image interpreter and included in the second input information, the diagnostician inputs information indicating approval of the second input information by putting a checkmark in a "Positive" check box on the display region C9 via the input device 24. On the other hand, in a case where the diagnostician disapproves the lesion found by the image interpreter and included in the second input information, the diagnostician inputs information indicating disapproval of the second input information by putting a checkmark in a "Negative" check box on the display region C9 via the input device 24.

In addition, the diagnostician inputs the comprehensive diagnosis result to the display region C6 via the input device 24. Examples of the comprehensive diagnosis result include a comprehensive diagnosis result related to a patient, which is obtained through interpretation of the medical image, such as a message saying "Since there is a suspicion of disease A, please consult at hospital B" and a message saying "There is no particular problem".

The reception unit 74 receives various types of information including the comprehensive diagnosis result input as described above. The transmission unit 76 transmits various types of information including the comprehensive diagnosis result received by the reception unit 74 to the shared server 16 via the network I/F 25. The shared server 16 stores the information transmitted from the information processing device 14. In other words, the transmission unit 76 stores the comprehensive diagnosis result received by the reception unit 74 in the shared server 16.

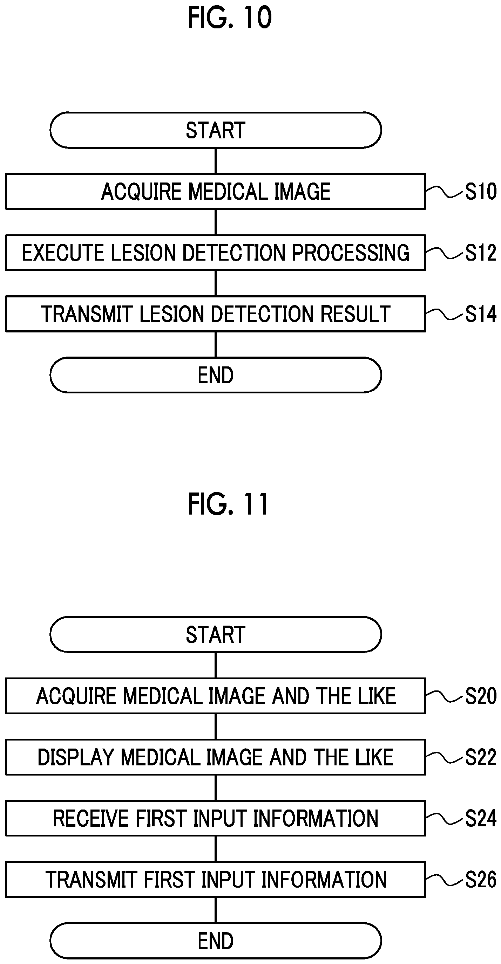

Next, an action of the information processing system 10 according to the present embodiment will be described with reference to Figs. 10 to 13. The CPU 20 of the information processing device 11 executes the information processing program 30, whereby first diagnosis support processing shown in Fig. 10 is executed. The first diagnosis support processing shown in Fig. 10 is executed, for example, in a case where an instruction to start execution is input by the imaging technician via the input device 24.

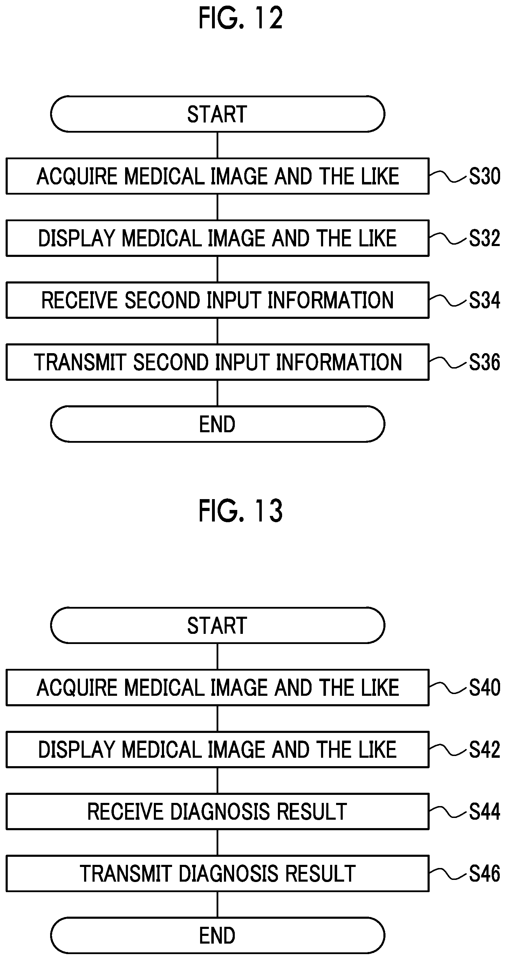

In addition, the CPU 20 of the information processing device 12 executes the information processing program 30, whereby second diagnosis support processing shown in Fig. 11 is executed. The second diagnosis support processing shown in Fig. 11 is executed, for example, in a case where an instruction to start execution is input by the imaging technician via the input device 24. Further, the CPU 20 of the information processing device 13 executes the information processing program 30, whereby third diagnosis support processing shown in Fig. 12 is executed. The third diagnosis support processing shown in Fig. 12 is executed, for example, in a case where an instruction to start execution is input by the image interpreter via the input device 24. Further, the CPU 20 of the information processing device 14 executes the information processing program 30, whereby fourth diagnosis support processing shown in Fig. 13 is executed. The fourth diagnosis support processing shown in Fig. 13 is executed, for example, in a case where an instruction to start execution is input by the diagnostician via the input device 24.

In step S10 of Fig. 10, the acquisition unit 40 acquires the medical image to be diagnosed from the image storage system 15 via the network I/F 25. In step S 12, the detection unit 42 executes the lesion detection processing on the medical image acquired in step S10. In step S14, the transmission unit 44 transmits the lesion detection result obtained by the processing of step S12 to the shared server 16 via the network I/F 25. In a case where the processing of step S14 ends, the first diagnosis support processing ends.

In step S20 of Fig. 11, the acquisition unit 50 acquires the medical image to be diagnosed from the image storage system 15 via the network I/F 25. In addition, the acquisition unit 50 acquires the lesion detection result corresponding to the medical image to be diagnosed from the shared server 16 via the network I/F 25.

In step S22, the display control unit 52 performs control to display the medical image and the lesion detection result, which are acquired in step S20, on the display 23. In step S24, as described above, the reception unit 54 receives the first input information of the imaging technician for the lesion detection result, which is input via the first display screen (see Fig. 5) displayed on the display 23 by the processing of step S22. In step S26, the transmission unit 56 transmits the first input information received in step S24 to the shared server 16 via the network I/F 25. In a case where the processing of step S26 ends, the second diagnosis support processing ends.

In step S30 of Fig. 12, the acquisition unit 60 acquires the medical image to be diagnosed from the image storage system 15 via the network I/F 25. In addition, the acquisition unit 60 acquires the lesion detection result and the first input information corresponding to the medical image to be diagnosed from the shared server 16 via the network I/F 25.

In step S32, the display control unit 62 performs control to display the medical image, the lesion detection result, and the first input information, which are acquired in step S30, on the display 23. In step S34, as described above, the reception unit 54 receives the lesion detection result and the second input information of the image interpreter for the first input information, which are input via the second display screen (see Fig. 7) displayed on the display 23 by the processing of step S32. In step S36, the transmission unit 66 transmits the second input information received in step S34 to the shared server 16 via the network I/F 25. In a case where the processing of step S36 ends, the third diagnosis support processing ends.

In step S40 of Fig. 13, the acquisition unit 70 acquires the medical image to be diagnosed from the image storage system 15 via the network I/F 25. In addition, the acquisition unit 70 acquires the lesion detection result and the second input information corresponding to the medical image to be diagnosed from the shared server 16 via the network I/F 25.

In step S42, the display control unit 72 performs control to display the medical image, the lesion detection result, and the second input information, which are acquired in step S40, on the display 23. In step S44, as described above, the reception unit 74 receives various types of information including the comprehensive diagnosis result, which is input via the third display screen (see Fig. 9) displayed on the display 23 by the processing of step S42. In step S46, the transmission unit 76 transmits the various types of information including the comprehensive diagnosis result, which is received in step S44, to the shared server 16 via the network I/F 25. In a case where the processing of step S46 ends, the fourth diagnosis support processing ends.

As described above, according to the present embodiment, a flow is realized in which the imaging technician, the image interpreter, and the diagnostician perform only a simple confirmation for the lesion detected by the computer and intensively confirm a region in which nothing is detected in the medical image by the computer. Accordingly, it is possible to improve the efficiency of diagnosis using the CAD result performed by three parties, that is, the imaging technician, the image interpreter, and the diagnostician.

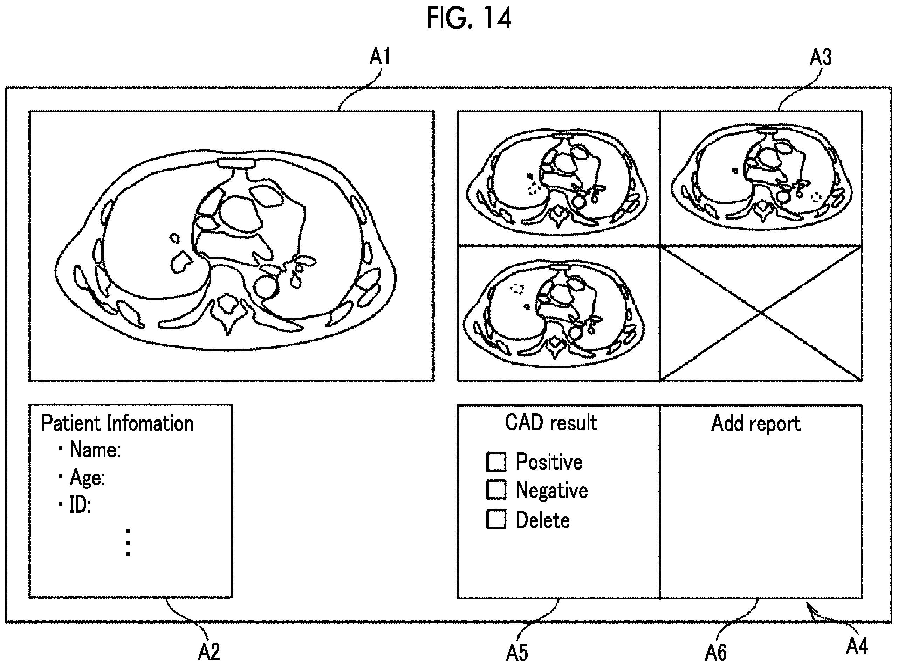

In the above-described embodiment, an aspect may be employed in which information indicating deletion of the lesion included in the lesion detection result is applied as the first input information and as the second input information. In this case, as shown in Fig. 14 as an example, the imaging technician inputs information indicating the deletion of the lesion included in the lesion detection result by putting a checkmark in a "Delete" check box on the display region A5 via the input device 24. Further, in this case, the shared server 16 deletes the information indicating the lesion from the lesion detection result.

In addition, in the above-described embodiment, the display control unit 72 may perform control to display the first input information on the display 23 in addition to the medical image and the second input information. In this case, for example, the same display region as the display region B9 on the second display screen is provided on the third display screen.

In addition, in the above-described embodiment, in a case where the lesion detection result includes information indicating one or more lesions, the display control unit 52, the display control unit 62, and the display control unit 72 may perform control to display the medical image to be diagnosed in a state in which the number of medical images is reduced, when displaying the medical image. In this case, for example, the display control unit 52, the display control unit 62, and the display control unit 72 perform control to display a tomographic image every other image when switching the display of the tomographic image, thereby halving the number of displayed medical images.

Further, in the above-described embodiment, for example, various processors shown below can be used as the hardware structure of a processing unit that executes various types of processing, such as each functional unit of the information processing devices 11 to 14. The above-described various processors include, for example, a programmable logic device (PLD) which is a processor having a changeable circuit configuration after manufacture, such as a field programmable gate array (FPGA), and a dedicated electrical circuit which is a processor having a dedicated circuit configuration designed for executing specific processing, such as an application specific integrated circuit (ASIC), in addition to the CPU which is a general-purpose processor that executes software (programs) to function as various processing units, as described above.

One processing unit may be composed of one of these various processors or a combination of two or more processors of the same type or different types (for example, a combination of a plurality of FPGAs or a combination of a CPU and an FPGA). Alternatively, a plurality of processing units may be composed of one processor.

A first example in which a plurality of processing units are composed of one processor is an aspect in which one or more CPUs and software are combined to constitute one processor and the processor functions as the plurality of processing units, as typified by a computer, such as a client and a server. A second example is an aspect in which a processor that realizes all the functions of a system including a plurality of processing units with one integrated circuit (IC) chip is used, as typified by a system on chip (SoC). As described above, various processing units are composed of one or more of the above-described various processors as the hardware structure.

Further, as the hardware structure of these various processors, more specifically, an electrical circuit (circuitry) in which circuit elements, such as semiconductor elements, are combined can be used.

In the above-described embodiment, an aspect in which the information processing program 30 is stored (installed) in advance in the storage unit 22 has been described, but the present disclosure is not limited thereto. The information processing program 30 may be provided in a form of being recorded on a recording medium, such as a compact disc read only memory (CD-ROM), a digital versatile disc read only memory (DVD-ROM), and a Universal Serial Bus (USB) memory. Alternatively, the information processing program 30 may be downloaded from an external device via the network.

### Explanation of References

10: information processing system
11, 12, 13, 14: information processing device
15: image storage system
16: shared server
20: CPU
21: memory
22: storage unit
23: display
24: input device
25: network I/F
27: bus
30: information processing program
40, 50, 60, 70: acquisition unit
42: detection unit
44, 56, 66, 76: transmission unit
52, 62, 72: display control unit
54, 64, 74: reception unit
A1 to A6, B1 to B9, C1 to C9: display region

## Claims

1. An information processing system comprising:
a first information processing device for diagnosis of a medical image performed by a computer;
a second information processing device for an imaging technician who captures the medical image;
a third information processing device for an image interpreter who interprets the medical image; and
a fourth information processing device for a diagnostician who performs a comprehensive diagnosis of a patient,
wherein each of the first information processing device, the second information processing device, the third information processing device, and the fourth information processing device includes at least one processor,
the processor of the first information processing device is configured to
acquire a medical image to be diagnosed,
execute lesion detection processing on the acquired medical image, and
transmit an execution result of the lesion detection processing,
the processor of the second information processing device is configured to
perform control to display the medical image and the execution result,
receive first input information of the imaging technician for the execution result, and
transmit the first input information,
the processor of the third information processing device is configured to
perform control to display the medical image, the execution result, and the first input information,
receive second input information of the image interpreter for the execution result, and
transmit the second input information, and
the processor of the fourth information processing device is configured to
perform control to display the medical image, the execution result, and the second input information,
receive a comprehensive diagnosis result of the diagnostician, and
store the diagnosis result.

2. The information processing system according to claim 1,
wherein the first input information is information indicating whether to approve or disapprove the execution result.

3. The information processing system according to claim 1 or 2,
wherein the first input information is information indicating a non-detected lesion that is not included in the execution result.

4. The information processing system according to any one of claims 1 to 3,
wherein the first input information is information indicating deletion of a lesion included in the execution result.

5. The information processing system according to any one of claims 1 to 4,
wherein the second input information is information indicating whether to approve or disapprove the execution result and the first input information.

6. The information processing system according to any one of claims 1 to 5,
wherein the second input information is information indicating a non-detected lesion that is not included in the execution result and in the first input information.

7. The information processing system according to any one of claims 1 to 6,
wherein the processor of the fourth information processing device is configured to
perform control to display the first input information in addition to the medical image and the second input information.

8. The information processing system according to any one of claims 1 to 7,
wherein there are a plurality of the medical images to be diagnosed, and
the processors of the second information processing device, of the third information processing device, and of the fourth information processing device are configured to
perform, in a case where the execution result includes information indicating one or more lesions, control to display the medical image in a state in which the number of medical images is reduced, when displaying the medical image to be diagnosed.

9. An information processing method using an information processing system including a first information processing device for diagnosis of a medical image performed by a computer, a second information processing device for an imaging technician who captures the medical image, a third information processing device for an image interpreter who interprets the medical image, and a fourth information processing device for a diagnostician who performs a comprehensive diagnosis of a patient, the information processing method comprising:
causing a processor provided in the first information processing device to
acquire a medical image to be diagnosed,
execute lesion detection processing on the acquired medical image, and
transmit an execution result of the lesion detection processing;
causing a processor provided in the second information processing device to
perform control to display the medical image and the execution result,
receive first input information of the imaging technician for the execution result, and
transmit the first input information;
causing a processor provided in the third information processing device to
perform control to display the medical image, the execution result, and the first input information,
receive second input information of the image interpreter for the execution result, and
transmit the second input information; and
causing a processor provided in the fourth information processing device to
perform control to display the medical image, the execution result, and the second input information,
receive a comprehensive diagnosis result of the diagnostician, and
store the diagnosis result.

10. An information processing program in an information processing system including a first information processing device for diagnosis of a medical image performed by a computer, a second information processing device for an imaging technician who captures the medical image, a third information processing device for an image interpreter who interprets the medical image, and a fourth information processing device for a diagnostician who performs a comprehensive diagnosis of a patient,
the information processing program causing a processor provided in the first information processing device to execute processing of:
acquiring a medical image to be diagnosed;
executing lesion detection processing on the acquired medical image; and
transmitting an execution result of the lesion detection processing,
causing a processor provided in the second information processing device to execute processing of:
performing control to display the medical image and the execution result;
receiving first input information of the imaging technician for the execution result; and
transmitting the first input information,
causing a processor provided in the third information processing device to execute processing of:
performing control to display the medical image, the execution result, and the first input information;
receiving second input information of the image interpreter for the execution result; and
transmitting the second input information, and
causing a processor provided in a processor of the fourth information processing device to execute processing of:
performing control to display the medical image, the execution result, and the second input information;
receiving a comprehensive diagnosis result of the diagnostician; and
storing the diagnosis result.
